# EUROPEAN PATENT SPECIFICATION

(11) **EP 2 561 890 B1**
(45) Date of publication and mention of the grant of the patent: **15.11.2017**
(21) Application number: 12005995.1
(22) Date of filing: 22.08.2012
(51) Int. Cl.: A61K 39/395

(54) **A method for producing medicaments for combatting tumors**
Verfahren zur Herstellung von Medikamenten zur Bekämpfung von Tumoren
Procédé pour produire des médicaments permettant de lutter contre les tumeurs

(30) Priority: 25.08.2011 DE 102011111631
(43) Date of publication of application: 27.02.2013
(60) Divisional application: 17001634.9
(73) Proprietor: Würfel, Wolfgang, 85235 Sixtnitgern (DE)
(72) Inventor: Würfel, Wolfgang, 85235 Sixtnitgern (DE)
(74) Representative: Samson & Partner Patentanwälte mbB

(56) References cited:
- JP-A- 2003 012 544
- KOMOHARA YOSHIHIRO ET AL: "HLA-G as a target molecule in specific immunotherapy against renal cell carcinoma", ONCOLOGY REPORTS, vol. 18, no. 6, December 2007 (2007-12), pages 1463-1468, XP002689652, ISSN: 1021-335X
- MATHEW MRUDULA ET AL: "Humanized immunotoxins: a new generation of immunotoxins for targeted cancer therapy", CANCER SCIENCE, JAPANESE CANCER ASSOCIATION, TOKYO, JP, vol. 100, no. 8, 1 August 2009 (2009-08-01), pages 1359-1365, XP002575095, ISSN: 1347-9032, DOI: 10.1111/J.1349-7006.2009.01192.X [retrieved on 2009-04-17]
- N. Rouas-Freiss: "HLA-G Proteins in Cancer: Do They Provide Tumor Cells with an Escape Mechanism?", Cancer research, vol. 65, no. 22, 15 November 2005 (2005-11-15), pages 10139-10144, XP55192264, ISSN: 0008-5472, DOI: 10.1158/0008-5472.CAN-05-0097
- V. Padler-Karavani ET AL: "Human Xeno-Autoantibodies against a Non-Human Sialic Acid Serve as Novel Serum Biomarkers and Immunotherapeutics in Cancer", Cancer research, vol. 71, no. 9, 1 May 2011 (2011-05-01), pages 3352-3363, XP055064496, ISSN: 0008-5472, DOI: 10.1158/0008-5472.CAN-10-4102
- XU HE ET AL: "HLA-G Expression in Human Breast Cancer: Implications for Diagnosis and Prognosis, and Effect on Allocytotoxic Lymphocyte Response After Hormone Treatment In Vitro", ANNALS OF SURGICAL ONCOLOGY, SPRINGER-VERLAG, NE, vol. 17, no. 5, 6 January 2010 (2010-01-06), pages 1459-1469, XP019785957, ISSN: 1534-4681
- MENIER C ET AL: "Human leukocyte antigen-G is expressed in advanced-stage ovarian carcinoma of high-grade histology", HUMAN IMMUNOLOGY, NEW YORK, NY, US, vol. 70, no. 12, 1 December 2009 (2009-12-01), pages 1006-1009, XP026750947, ISSN: 0198-8859, DOI: 10.1016/J.HUMIMM.2009.07.021 [retrieved on 2009-08-04]

## Description

### Field of the Invention

The invention relates to methods of producing medicaments for combatting tumors and to the use thereof for said purpose.

### Background of the Invention

The chemical or biological tumor therapy has not yet focused on individual tumor cell types of a single patient but still detects in general all rapidly dividing cells, independent of whether or not they are malignant and independent of the individual patient.

In the final result, this type of therapy is often ineffective and/or accompanied by severe side-effects. A more targeted, highly individual therapy would be extremely useful.

The basis for the well-regulated existence of an entire organism is the communication between the cells or the cellular dialogue. This dialogue and its regulation enable to maintain the existence of an entire organism even though cells constantly die and/or are reproduced. As a result of this dialogue, the differentiation of cells is also regulated, as known from stem cell research. This dialogue even enables the well-regulated cooperation of two different cell clones even if one shows an extremely fast growth as is the case during pregnancy.

The basis of the cellular dialogue in man is the MHC (major histocompatibility complex) with its HLA groups. The identification of a cell by HLA groups is the basis of every cellular communication. The cellular communication in which develops in cooperation with specific receptors, such as the killer-immunoglobulin-like receptors (KIR) on the natural killer cells (NK cells) or the LILR (leukocyte immunoglobulin-like receptors), wit subsequent involvement of further factors, such as cytokines, growth factors, etc.

Various HLA groups are known which can be described as follows:

HLA groups A, B, C and C (MHC I): they identify substantially all adult and somatic cells.

HLA groups D (DR, DP, DQ, etc.; MHC II): They play an important role in immunocompetent cells and/or in the antigen presentation.

HLA groups E, F and G: They identify embryonic cells, in particular on the so-called front of invasion.

HLA group H and the following: The function of these so-called "pseudo-antigens" is not yet quite clear.

In addition, the MHC complex also comprises further substances such as the complement factors which belong to class III.

A tumor cell basically has a genetic code the same as that of any other cell of the entire organism. Therefore, it does not have any information other than that of the entire organism with respect to cell division and cell differentiation. As a result, every malignant tumor disease is unique and individual, i.e. specific to the respective organism.

In some tumor diseases, additional genetic information is introduced into the cell from outside, e.g. by viral vectors, such as the so-called oncogenes. However, oncogenes can also form an integral part of the genetic material from procreation on. Oncogenes and/or the activation thereof and other external factors can permanently affect the biology of a tumor cell. Yet, the tumor cells stay involved in the cellular dialogue of the entire organism and the regularities valid therein.

Organisms having a high cellular differentiation, such as human beings, "pay" for their high differentiation by a loss of multipotency or totipotency. In the case of organ losses, *restitutio ad integrum* is no longer possible but only the repair by connective tissue. When a living being is not so strongly differentiated, such as the starfish, the loss of totipotency or multipotency is not so distinct and therefore when an arm is lost, for example, a new arm can grow again even though it is smaller.

Totipotency is basically encoded in the genetic material of higher living beings as well. This is proved by the simple fact that this genetic material formerly had to control the development from a fertilized egg cell to a differentiated organism. Cloning experiments also show that "resetting" of the genetic material ("reprogramming") of even highly differentiated cells, such as the udder cells of the cloned sheep "Dolly", "to zero" is possible in the nucleus. In the final analysis, this also applies to the procreation and/or fertilization of an egg cell where the genetic material of two relatively old individuals (father and mother to be) is reset "to zero" and is encoded again for the development of a new living being.

Correspondingly, it is clear that a tumor cell is also provided with genetic material that fundamentally codes for all growth and differentiation processes which are at all possible in an entire organism, i.e. also for the mechanisms of the initial embryonic implantation, of the early embryo-maternal cell dialog and of the subsequent embryonic-fetal development.

Every tumor cell loses its differentiation in varying degrees (is thus "dedifferentiated") and makes its "way back" in varying degrees. Essential characteristics of this "way back" are the loss of cellular differentiation and the loss of specific cell performances and also the (re)gaining of uncontrolled cell growth.

It is known that tumor cells can express typical embryonic HLA groups on their surface. Although the respective investigations are still fragmentary, it is postulated here that this expression of embryonic HLA groups (namely the known ones and optionally also not yet identified ones, e.g. in the scope of so-called "pseudogenes") contributes to the circumstance that tumor cells evade the attack of the unspecific immune defense of the own organism. The expression of these typical HLA groups on the surface enables cells to activate corresponding receptors e.g. on the NK cells but also the lymphocytes and further immunocompetent cells, and therefore there is not only no attack of the unspecific immune defense, i.e. the NK cells and lymphocytes, but also in the individual case tumor cells (and also embryonic cells) are able to "let the immune defense work for them", namely by a synthesis of growth factors and cytokines which are beneficial to the own development.

Komohara et al., (Oncology Reports 18(6), 1463-68, 2007) for instance discloses HLA-G specific peptides for inducing specific CTLs are cytotoxic against a renal cell carcinoma (RCC) cell line. The authors discuss that HLA-G could be a target antigen in specific immunotherapy against HLA-G expressing RCC and other HLA-G expressing cancers. Here, e.g. the phenomenon of TAM (tumor-associated macrophages) or MDSC (tolerogenic "myeloid-derived suppressor cells") has to be mentioned. They even support the tumor growth in the micro-environment of a malignant tumor (i.e. "are turned"). The same applies similarly to cytokines, such as MIF (macrophage migration inhibition factor) which is likely to be produced in the tumor (likely by NK cells) and has a proangiogenetic effect, thus supporting the proliferation and migration of tumor cells.
Tumor cells need not be very resistant. As is known, they are more sensitive to chemotherapy and also more sensitive to radiation than "healthy" and differentiated standard cells. Their cell division rate is not particularly high either.
The danger resulting from a malignant tumor cell is above all that it is able on the basis of the cellular communication to "enforce" the progressing uncontrolled growth.
This circumstance is also illustrated by the fact that according to current knowledge metastases predominantly form because malignant cells which can be referred to as "malignant stem cells" spread and colonize. Should this be correct, such "malignant stem cells" should by means of the cellular communication with the adjacent tissue also be locally able to evade the growth control and the differentiation pressure. Even if formed from a dedifferentiation, stem cells would behave like stem cells in general, which in this case directs the focus in particular on the mode of functioning of the embryomaternal communication (of the embryomaternal dialogue).

The malignant degeneration of a cell is a unique process which is specific to every individual. This is not altered by the fact that there are pathologically well classifiable (always recurring) tumor types across individuals. This circumstance is rather a proof for the fact that a malignant tumor does not form by every dedifferentiation and every "way back". It is rather likely that only certain constellations can "survive" on the "way back", thus resulting in the typical tumor entities across individuals.

The dedifferentiation or "degeneration" of a cell is presumably a comparatively ubiquitous process in every entire organism. However, it almost never leads to the formation of a tumor disease since only some few of these cells have the cell-biological and also communicative preconditions (from cell to cell) that are necessary for the survivability. The cells which show survivability use the two above mentioned mechanisms, presumably in a combined form. On the one hand, they use the recovery of the embryomaternal communication to evade an attack of the immune system and even get support from this communication in the course of cell growth; on the other hand, they can evade an attack of the specific immune defense while being protected by the completely or partially expressed (originally adult) HLA patterns (and also those which correspond to those of the own mother (see below)), thus expanding.

The "acquired" immunity develops during pregnancy and means that there is not only a tolerance with respect to the body's own HLA groups but also always with respect to the foreign adult HLA groups of one's own mother.

A tumor cell basically expresses the same adult HLA groups as all other somatic cells of the entire organism. As a result, it is protected from an attack of the specific immune defense. This also applies in principle when parts of the original HLA pattern are lost "on their way back", are expressed less densely or are available in a changed, i.e. corrupted, form (which is not atypical for tumor cells).

It is here useful to make a comparison with an embryo's embryonic cells which were flushed out in the maternal organism and persist therein (this is referred to as "microchimerism). The embryonic surface structures, in particular HLA-G, -E and -F, on the embryonic cells (predominantly placental or trophoblastoid) prevent the mother's immune system from attacking the cells.

If a certain differentiation of the embryonic cells has already taken place (here usually the embryo's genuine cells), they are specifically incorporated into corresponding organs. This very strongly reflects the behavior of malignant tumors which often prefer a certain metastasis formation pattern.

When the embryonic surface structures of the embryo cells are maintained, the embryonic cells are presumably not attacked throughout the mother's life - similar to tumor cells.

If during pregnancy very many embryonic cells flush out into the maternal organism, this tolerance can even lead to an attempt to "take over" the mother's body, i.e. a "graft-versus-host" reaction results, as in the case of the quite threatening HELLP syndrome (hemolysis, elevated liver enzymes, low platelet count), for example.

However, there can also be counter-reactions to microchimerism, which are presumably due to the fact that the embryonic cells at least partially lose their HLA-G, E, F protection, optionally accompanied by further differentiation.

Then, there are inflammatory counter-reactions of the maternal organism since the embryonic cells show "adult" HLA structures when differentiating which differ from those of the maternal organism. As a typical result, connective tissue forms around the site of inflammation and this can cause certain immune diseases (cf. Hashimoto's thyreoiditis).

This inflammatory counter-reaction of the organism does not take place in the case of tumor cells since the tumor cells form HLA characteristics on the surface during further differentiation, said characteristics not differing from those of the host organism.

This complete or incomplete expression of the adult HLA patterns prevents an attack of the specific immune defense even when the tumor cell stands out by additional antigen expressions (or overexpressions) as known and described for tumor cells. The protection resulting from the complete or incomplete expression of the original adult HLA patterns is obviously very effective such that a tumor cell can express its specific antigen patterns (thus "standing out" as a result) without an effective attack of the (specific) immune response, i.e. also of the B lymphocytes and T lymphocytes occurring. It is remarkable that the tumor antigen expression patterns are relatively specific to individual tumor types. It is also possible that changed and/or mutated MHC-/HLA groups make the antigen presentation cascade (APM (antigen processing machinery)) more and more faulty and therefore typical, human-associated or own antigens are hardly presented or are not presented (any more).

When an entire organism is confronted with a somatic cell whose HLA expression pattern strongly or fully differs from that of the organism or the mother, it has to be assumed that there will be an attack of the immune defense accompanied by the formation of memory cells. The "memory" is, of course, predominantly directed against divergent HLA groups. However, it is known that such an attack of the immune system followed by a destruction of the foreign cells is also accompanied by the formation of antibodies against other surface structures. In individual cases, antibodies are even formed against ubiquitous cell constituents, such as phospholipids ("antiphospholipid syndrome").

Against this background, it is necessary to consider both mechanisms as an entirety, namely as an entirety of how the "tumor cell on its way back" communicates with the other cells and the immune system and/or how lost communication characteristics of the embryonic period are recovered so as to develop from this understanding a therapy concept considering these basic principles and corresponding medicaments.

Besides the specific immune defense (i.e. T- and B-lymphocytes), monocytes and the macrophages resulting from them also play a role in tumor growth. However, macrophages can only be activated when cells of the unspecific immune defense (such as NK killer cells) or the specific immune defense (such as T-cells or B-cells) are present. However, this requires "priming" where antigen-presenting cells (such as the dendritic cells) present mutated or "foreign" proteins, thus leading to the formation of cytotoxic T-cells. Here, numerous cytokines, such as interferon γ (IFN-γ) or the tumor necrosis factor (TNF-α) play a role as well.
A comparison between embryonic cells and tumor cells offers itself in the case of the macrophages as well. Macrophages can be found in the basal endometrium in the case of an establishing pregnancy. They usually have an inhibitory effect on the invasion behavior of the embryo and form so to speak a "protective wall" between the implanting embryo and the myometrium. On the other hand, the embryo secretes macrophage migration inhibiting factors, i.e. factors which limit and inhibit the attack of the macrophages. This applies likewise to malignant tumors (see above)

### Summary of the invention

In a first aspect, the invention relates to a method of producing a medicament for combatting a tumor in a host, comprising determining the individual embryonic HLA expression pattern of a tumor cell of the tumor in a sample from the patient, the HLA expression pattern including expression of HLA-E, HLA-F, and HLA-G class I minor genes, developing antibodies against the expression pattern and coupling the antibodies with a cytotoxin, such as a chemotherapeutic agent or a radioisotope, wherein the thus created individual antibodies serve as an antitumor medicament and/or the antibodies are to be used to destroy the tumor-specific embryonic HLA pattern.

A second aspect of the invention relates to antibodies produced as described above, for use in combatting a tumour. A third aspect, which is not covered by the claims, relates to a method of producing a medicament for combatting a tumor in a host, comprising removing tumor cells with an individual expression pattern and exposing them *in vitro* to immunocompetent cells, preferably of the host, thus "training" the immunocompetent cells against the individual expression pattern and using the thus trained cells as an antitumor medicament for the *in vivo* introduction into the host.

A fourth aspect, which is not covered by the claims, relates to the use of a medicament which is produced as described in the third aspect for combatting a tumor.

A fifth aspect, which is not covered by the claims, relates to a method of producing a medicament for combatting a tumor in a host, wherein heterologous tumor cells are selected which have tumor-specific antigen patterns the same or similar to those of the tumor cells of the host, but have adult HLA groups completely differing therefrom (and - if possible - from the mother), the selected heterologous tumor cells are freed, i.e. are denuded, from the embryonic HLA expression pattern by means of a medicament produced according to the first aspect, and killed and the resulting heterologous tumor cells serve as an antitumor medicament for introduction into a host.

A sixth aspect, which is not covered by the claims, relates to the use of a medicament which is produced as described in the fifth aspect for combatting a tumor.

A seventh aspect, which is not covered by the claims, relates to a method of producing an antitumor antibody, wherein with respect to a host heterologous tumor cells are selected which have tumor-specific antigen patterns the same or similar to those of the tumor cells of the host, but have adult HLA groups fully differing therefrom (and from the mother), the selected heterologous tumor cells are freed, i.e. denuded, from the embryonic HLA expression pattern by means of a medicament produced according to the first aspect and are exposed optionally in the presence of a complete or incomplete adjuvant to immunocompetent cells, preferably of the host, whereby the immunocompetent cells are trained against both the different adult HLA group antigens and the tumor-specific antigens so as to express tumor-specific antibodies, and the antibodies directed against the tumor-specific antigen are isolated.
An eighth aspect, which is not covered by the claims, relates to the use of the antibody produced by the method of the seventh aspect as an antitumor medicament.
A ninth aspect, which is not covered by the claims, relates to a method of producing genetically modified immunocompetent cells, in particular genetically modified NK cells and also genetically modified lymphocytes, comprising silencing the genes for the receptors which convey an inhibitory effect and are activated by the tumor's own HLA groups, whether they are adult (MLA-A, -B, -C, -D) or embryonic (HLA-E, -F, -G *inter alia*). The cells can be proliferated *in vitro.* A tenth aspect, which is not covered by the claims, relates to immunocompetent cells which lack the receptors conveying an inhibitory effect which are activated by the tumor's own HLA groups, whether they are adult (MLA-A, -B, -C, -D) or embryonic (HLA-E-, -F, -G *inter alia*). Thus, no inhibition can be exerted by the tumors. In particular, NK cells are concerned here whose genes for the receptors which convey an inhibitory effect and which recognize the body's own HLA-A, -B, -C and/or -D are deleted.
An eleventh aspect, which is not covered by the claims, relates to the use of the cells according to the tenth aspect as a medicament for combatting a tumor.

### Detailed description

A first aspect of the invention relates to a method of producing a medicament for combatting a tumor in a host, comprising determining the individual embryonic HLA expression pattern of a tumor cell of the tumor in a sample from the patient, the HLA expression pattern including expression of HLA-E, HLA-F, and HLA-G class I minor genes, developing antibodies against the expression pattern and coupling the antibodies with a cytotoxin, such as a chemotherapeutic agent or a radioisotope, wherein the thus created individual antibodies serve as an antitumor medicament and/or the antibodies are to be used to destroy the tumor-specific embryonic HLA pattern.

A second aspect of the invention relates to antibodies produced as described above, for use in combatting a tumor. The partial or full recovery of embryonic HLA expression patterns on malignant cells can serve for identifying and combatting these malignant cells.
As already mentioned above, malignant tumor cells regain the embryonic HLA expression patterns on their "way back" in varying degrees. These are predominantly the HLA groups F and G but also groups E and others and possibly also some pseudoantigens, i.e. HLA groups H and higher. As already mentioned above, it is characteristic of these embryonic HLA groups that they are not only able to interact e.g. with specific receptors on NK cells (NKG2-/KL (killer lecithin-like receptors) or the KIR formations or the lymphocytes (LIL receptors) and/or immunoglobulin-like transcripts (ILT) but that these interactions besides a "blockade" of the immune system also lead as to the fact that various immunocompetent cells (e.g. NK cells and macrophages) synthesize and secrete cell growth- and cell division-promoting cytokines and growth factors. As a result, it is possible for the tumor cell to manipulate the cellular dialogue so as to enable unlimited cell growth. In addition, it is also possible in individual cases that the secreted cytokines and growth factors support this uncontrolled cell growth and/or cell division behavior.
Especially in the case of stem cells which usually do not express any adult HLA groups (A to D) and therefore are especially a target of the unspecific immune defense, i.e. the NK cells and the macrophages, this mechanism is of major significance.

There are already publications which show that the malignancy of a tumor correlates with the increase in the HLA-G expression.

Since the regression of a tumor cell individually proceeds differently, it is necessary to determine the individual embryonic HLA expression pattern of a tumor cell (i.e. of HLA-F and G, also of HLA-E and optionally the pseudoantigens), which is nowadays rather easily possible. Once such an expression pattern is known, the development of specific antibodies is possible.

Since there are also reports that other tissues partially express embryonic groups, such as HLA-F or also HLA-G, it is important to detect the complex individual expression pattern of a tumor cell and develop thereagainst an antibody as specific as possible, i.e. a complex antibody, to avoid cross-reactions with other tissues. It is basically also conceivable to "incorporate" into these antibodies epitopes against other characteristics of a tumor cell, namely e.g. against typical tumor antigens which were detected on a certain, individual-specific tumor. It will be the simplest to achieve this by polyclonal antibodies. However, the use of a group of monoclonal antibodies, which are optionally coupled, is also possible.

The attachment of a polyclonal antibody or cross-linked monoclonal antibodies to an individual-specific tumor cell can now be utilized in various ways.

On the one hand, it is possible to couple e.g. chemotherapeutic agents or radioisotopes to such antibodies to thus damage the tumor cell.

A second possibility is to destroy the embryonic HLA patterns in this way, e.g. by combining the antibodies with enzyme complexes which directly attack HLA antigens or at least the scaffold proteins of the HLA complex. Furthermore, it would also be possible to "mask" the entire embryonic HLA complex by means of these antibodies such that it can no longer be recognized as such by the immune defense. As a result, the above described mechanisms of the "dialogue" would be omitted.

The implementation of the described measures does not have to take place *in vivo.* It is also basically possible to remove tumor cells and to "train" immunocompetent cells - after removal or masking of the embryonic HLA structures -by means of them *in vitro.*

Correspondingly, a third aspect, which is not covered by the claims, relates to a method of producing a medicament for combatting a tumor in a host, comprising removing tumor cells with an individual expression pattern and exposing them *in vitro* to immunocompetent cells, preferably of the host. As a result, the immunocompetent cells are trained against the individual expression pattern, and the thus trained cells serve as an antitumor medicament for *in vivo* introduction into the host.

A fourth aspect, which is not covered by the claims, is the use of a medicament which is produced as described in the third aspect for combatting a tumor.

The *"in vitro* training" offers the advantage of "training" the individual cell types of the unspecific and specific immune defense in controlled fashion by means of the specific HLA and tumor antigen expression patterns so as to be able to better dose the effect when these cells are returned to the original organism. However, as pointed out above, for this end the typical embryonic expression patterns must be removed or "masked" *in vitro,* but this also applies to the adult expression patterns (HL groups A to C and/or D and the possibly existing HLA groups which correspond to those of the own mother), since the immunocompetent cells of the specific immune defense, such as T-lymphocytes, can hardly or only poorly attack cells which have the typical adult expression patterns of the original organism.

In so far, the *in vitro* "training" of the immunocompetent cells of the specific and unspecific immune defense serves predominantly for training them against the individual-specific tumor antigen expression patterns -and this after the discontinuation of the blockade by the embryonic and adult HLA groups.

The adult HLA patterns of the original organism which are also fully or partially expressed by the tumor cells, thus represent the second effective protection of a tumor cell against an attack of the body's own immune defense, namely the cells of the specific immune defense.

A fifth aspect, which is not covered by the claims, thus relates to a method of producing a medicament for combatting a tumor in a host, wherein heterologous tumor cells are selected which have tumor-specific antigen patterns that are equal or similar to those of the tumor cells of the host but have adult HLA groups completely differing therefrom, the selected heterologous tumor cells are freed, i.e. denuded, from the embryonic HLA expression pattern by means of a medicament produced according to the first aspect, and are killed and the resulting killed heterologous tumor cells serve as an antitumor medicament, optionally together with an adjuvant, e.g. aluminum hydroxide or an oil-in-water emulsion, which comprises squalene, polysorbate 80 and vitamin E, for introduction into the host.

A sixth aspect, which is not covered by the claims, is the use of a medicament which is produced as described in the fifth aspect and optionally contains an adjuvant, for combatting a tumor.

Tumor cells which in the model case have a tumor-specific antigen expression pattern the same as that of the host organism however fully differ therefrom as regards the HLA groups, induce an immune response against these tumor cells in the host, namely on account of the divergent HLA expression patterns. In a response to the divergent HLA patterns, the specific immune defense will also "learn" the tumor antigen expression patterns and form antibodies and cells of the cellular immune defense which are directed thereagainst. The immunological response, i.e. the formation of antibodies and/or the cellular immune response to the tumor-specific antigen expressions, can be promoted by adjuvants, such as aluminum hydroxide or an oil-in-water emulsion which comprises squalene, polysorbate 80 and vitamin E. The immune response can also be evoked *in vitro* (*in vitro* "training" of the immunocompetent cells). The antibodies and the trained immunocompetent cells can then be used *in vivo* or *in vitro* to attack the individual-specific tumor cells.

As already pointed out, the adult HLA groups (A to D) which are fully or partially left on the surface protect a tumor cell against an attack of the specific immune defense. In order to carry out a sensitization of the immune defense against the typical surface expression patterns of a tumor cell, it is thus necessary to supply killed tumor cells with oncogenes as identical as possible and an HLA expression pattern as divergent as possible.

The difficulty is to identify tumor cells being as HLA-A to D-divergent as possible and having tumor-specific antigen patterns as equal as possible (as in the host organism). While it is relatively easy to determine the HLA patterns, this is markedly more difficult with the typically tumor-specific antigen expressions. Here, only a "constriction" can be made for the time being, namely by means of the greatest possible number of different (known) antigen expression patterns. Thus, there is a residual insecurity which can, however, be avoided by basing the therapy on different tumor cell lines having HLA patterns as discrepant as possible and tumor antigen expression patterns as similar as possible.

This therapeutic approach would be facilitated by establishing "tumor cell banks".

It is also necessary for the heterologous tumor cells not to have any embryonic HLA groups and/or features; therefore, they also have to be denuded.

It might be that in future genetic engineering methods also offer a possibility here: for example, the introduction of a genome of a tumor cell into a cell as HLA-divergent as possible is conceivable (e.g. by cloning), however, with the proviso that the adult (and if possibly also embryonic) HLA-gene loci of the tumor cell are silenced so as to finally form a hybrid cell which expresses divergent HLA patterns, but expresses the typical tumor antigen patterns of the tumor cell of the original organism.

When the killed tumor cells which are as described above and which originate from a foreign person are injected, an immune response against these tumor cells must naturally occur in the case of an existing adult HLA expression. It has to be assumed that on this occasion memory cells against the other surface antigen structures are also formed, thus also against structures which are specific to this tumor. Once such cells have been formed, they will naturally turn against the similar surface antigen structures of the own tumor, i.e. against the own tumor cells. In this way, an autoimmune response against the body's own tumor cells is triggered. (Heterologous tumor cells induced autosensitization = HETINAS)

In order to counteract the variability of the tumor antigen expressions, it would even make sense to collect tumor cells from several person having divergent adult HLA patterns and inject them as a mix. The therapy can be used again and again situatively even if the tumor changes its differentiation or if metastases having a different surface antigen structure should form. What is decisive is always that the foreign cells in the adult HLA groups are divergent. However, this can be readily detected.

A seventh aspect, which is not covered by the claims, further relates to a method of producing an antitumor-antibody, wherein with respect to a host heterologous tumor cells are selected which have tumor-specific antigen patterns equal or similar to the tumor cells of the host, but have adult HLA groups which fully differ therefrom, the selected heterologous tumor cells are freed, i.e. denuded, from the embryonic HLA expression pattern by means of a medicament produced according to the first aspect of the invention and are exposed to immunocompetent cells, preferably of the host, optionally in the presence of an adjuvant so as to train the immunocompetent cells against both the different adult HLA group antigens and the tumor-specific antigens and in this connection express tumor-specific antigens, and the antibodies directed against the tumor-specific antigen are isolated.

An eighth aspect relates to the use of the antibody produced by the above method as an antitumor medicament.

A ninth aspect, which is not covered by the claims, relates to a method of producing genetically modified immunocompetent cells, in particular NK cells, comprising silencing the genes for the receptors which convey an inhibitory effect and are activated by the tumor's own HLA groups. Here, it must be determined individual-specifically in advance what HLA features the individual tumor uses to activate what receptors to then eliminate *in vitro* the genes for precisely these receptors.

A tenth aspect, which is not covered by the claims, relates to the thus produced immunocompetent cells, in particular NK cells.

An eleventh aspect, which is not covered by the claims, relates to the use of the cells of the tenth aspect as a medicament for combatting a tumor.

## Claims

1. A method of producing a medicament for combatting a tumor in a host, comprising
- determining the individual embryonic HLA expression pattern of a tumor cell of the tumor in a sample from the patient, the HLA expression pattern including expression of HLA-E, HLA-F, and HLA-G class I minor genes,
- developing antibodies against the expression pattern and
- coupling the antibodies with a cytotoxin, such as a chemotherapeutic agent or a radioisotope, wherein
the thus created individual antibodies serve as an antitumor medicament and/or the antibodies are to be used to destroy the tumor-specific embryonic HLA pattern.

2. Antibodies, which are produced as described in claim 1, for use in combatting a tumor.

## Patentansprüche

1. Verfahren zur Herstellung eines Medikaments zur Bekämpfung eines Tumors in einem Wirt, umfassend:
- Bestimmen des individuellen embryonalen HLA-Expressionsmusters einer Tumorzelle des Tumors in einer Probe von dem Patienten, wobei das HLA-Expressionsmuster Expression von HLA-E, HLA-F und HLA-G Klasse-I-Genen umfasst,
- Entwickeln von Antikörpern gegen das Expressionsmuster,
- Koppeln der Antikörper mit einem Zellgift, wie einem Chemotherapeutikum oder einem Radioisotop, wobei die so erstellten, individuellen Antikörper als Antitumor-Medikament dienen und/oder die Antikörper dazu verwendet werden sollen, das tumorspezifische embryonale HLA-Muster zu zerstören.

2. Antikörper, hergestellt wie im Anspruch 1 beschrieben, zur Verwendung bei der Bekämpfung eines Tumors.

## Revendications

1. Procédé de production d'un médicament pour combattre une tumeur chez un hôte, comprenant
- la détermination du profil d'expression HLA embryonnaire individuel d'une cellule tumorale de la tumeur dans un échantillon provenant du patient, le profil d'expression HLA comprenant l'expression des gènes mineurs HLA-E, HLA-F, et HLA-G de classe I,
- le développement d'anticorps dirigés contre le profil d'expression et
- le couplage des anticorps avec une cytotoxine, telle qu'un agent chimiothérapique ou un radio-isotope,
dans lequel les anticorps individuels ainsi créés servent de médicament antitumoral et/ou les anticorps sont destinés à être utilisés pour détruire le profil HLA embryonnaire spécifique de la tumeur.

2. Anticorps, qui sont produits comme décrit dans la revendication 1, destinés à être utilisés pour combattre une tumeur.
